# EUROPEAN PATENT SPECIFICATION

(11) **EP 4 461 348 B1**
(45) Date of publication and mention of the grant of the patent: **20.05.2026**
(21) Application number: 24174868.0
(22) Date of filing: 08.05.2024
(51) Int. Cl.: A61N 5/10

(54) **MEDICAL DEVICES AND MEDICAL SYSTEMS**
MEDIZINISCHE VORRICHTUNGEN UND MEDIZINISCHE SYSTEME
DISPOSITIFS MÉDICAUX ET SYSTÈMES MÉDICAUX

(30) Priority: 12.05.2023 CN 202310540187
(43) Date of publication of application: 13.11.2024
(73) Proprietor: Shanghai United Imaging Healthcare Co., Ltd., Shanghai 201807 (CN)
(72) Inventor: Ruan, Jianbin, Shanghai, 201807 (CN); Li, Xiaobin, Shanghai, 201807 (CN); Zhang, Jian, Shanghai, 201807 (CN)
(74) Representative: Wang, Bo

(56) References cited:
- US-A1- 2004 184 579
- US-A1- 2018 056 090
- US-A1- 2018 304 098

## Description

### TECHNICAL FIELD

The present disclosure relates to the technical field of medical devices, and in particular to a radiation imaging and treatment device and system.

### BACKGROUND

A radiation imaging and treatment device is a medical device that integrates both the medical scanning function and the radiotherapy function. The radiation imaging and treatment device uses a scanning device to scan a lesion site of a patient to assist a treatment device to accurately localize the lesion site. However, the treatment device and the scanning device of the current radiation imaging and treatment device can only irradiate the patient in a small angle range, the current radiation imaging and treatment device is not applicable to complex application scenarios. In addition, during the working process of the radiation imaging and treatment device, the treatment device and the scanning device need to be controlled to rotate around the patient and may interfere with each other or with the patient in the rotation process, which may affect the use safety of the radiation imaging and treatment device.

In order to solve the problems, the present disclosure provides a medical device and a medical system for improving the efficiency and accuracy of radiation imaging and treatment.
US 2018/304098 A1 relates to a radiation system that includes a radiation source providing therapeutic radiation, a first gantry supporting a second gantry carrying the radiation source.

### SUMMARY

The invention is defined in the appended set of claims.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities, and combinations set forth in the detailed examples discussed below.

### BRIEF DESCRIPTION OF THE DRAWINGS

The present disclosure will be further illustrated by way of exemplary embodiments, which will be described in detail with reference to the accompanying drawings. These embodiments are not limiting, and in these embodiments, the same numbering denotes the same structure, wherein:
FIG. 1 is a schematic diagram illustrating an exemplary medical device according to some embodiments of the present disclosure;
FIG. 2 is a schematic diagram illustrating an exemplary first support member of a medical device in FIG. 1 after rotation;
FIG. 3 is a schematic diagram illustrating an exemplary first support member of a medical device in FIG. 1 after rotation;
FIG. 4 is a schematic diagram illustrating an exemplary irradiation angle of a treatment device according to some embodiments of the present disclosure;
FIG. 5 is a schematic diagram illustrating an exemplary irradiation model of a treatment device according to some embodiments of the present disclosure;
FIG. 6 is a schematic diagram illustrating an exemplary medical device according to some embodiments of the present disclosure;
FIG. 7 is a schematic diagram illustrating an exemplary medical device according to some embodiments of the present disclosure;
FIG. 8 is a schematic diagram illustrating an exemplary medical device according to some embodiments of the present disclosure;
FIG. 9 is a schematic diagram illustrating an exemplary medical device according to some embodiments of the present disclosure; and
FIG. 10 is a schematic module diagram illustrating an exemplary medical system according to some embodiments of the present disclosure.

### DETAILED DESCRIPTION

In the following detailed description, numerous specific details are set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high level, without detail, in order to avoid unnecessarily obscuring aspects of the present disclosure. Various modifications to the disclosed embodiments will be readily apparent to those skilled in the art without departing from the scope of the present disclosure. Thus, the present disclosure is not limited to the embodiments shown, but to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this specification, specify the presence of stated features, integers, steps, operations, elements, and/or components, but do not preclude the presence or addition of one or more other features, integers, steps, operations, elements, components, and/or groups thereof.

It will be understood that the terms "system," "engine," "unit," "module," and/or "block" used herein are one method to distinguish different components, elements, parts, sections or assemblies of different levels in ascending order. However, the terms may be displaced by another expression if they achieve the same purpose.

It will be understood that when a unit, engine, module, or block is referred to as being "on," "connected to," or "coupled to," another unit, engine, module, or block, it may be directly on, connected or coupled to, or communicate with the other unit, engine, module, or block, or an intervening unit, engine, module, or block may be present, unless the context clearly indicates otherwise. As used herein, the term "and/or" includes any and all combinations of one or more of the associated listed items.

It will be understood that, although the terms "first," "second," "third," "fourth," etc., may be used herein to describe various elements, these elements should not be limited by these terms. These terms are only used to distinguish one element from another. For example, a first element could be termed a second element, and, similarly, a second element could be termed a first element, without departing from the scope of example embodiments of the present invention.

Spatial and functional relationships between elements (for example, between crystal elements) are described using various terms, including "connected," "engaged," "interfaced," and "coupled." Unless explicitly described as being "direct," when a relationship between first and second elements is described in the present disclosure, that relationship includes a direct relationship where no other intervening elements are present between the first and second elements, and also an indirect relationship where one or more intervening elements are present (either spatially or functionally) between the first and second elements. In contrast, when an element is referred to as being "directly" connected, engaged, interfaced, or coupled to another element, there are no intervening elements present. Other words used to describe the relationship between elements should be interpreted in a like fashion (e.g., "between," versus "directly between," "adjacent," versus "directly adjacent," etc.). As used herein, the term "and/or" includes any and all combinations of one or more of the associated listed items.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present disclosure. It is understood that the drawings are not to scale.

A radiation imaging and treatment device is a medical device that integrates both the medical scanning function and the radiotherapy function. The radiation imaging and treatment device uses a scanning device to scan a lesion site of a patient to assist a treatment device to accurately localize the lesion site. In some embodiments, ray emitters of the scanning device and the treatment device may emit scanning rays and treatment rays to the patient, respectively, and the scanning rays and treatment rays may penetrate through the patient to be received by corresponding receivers. In some embodiments, in order to realize multi-angle and multi-directional imaging and treatment needs, the scanning device and the treatment device may rotate around the lesion site during the working process. In order to ensure the use safety of the radiation imaging and treatment device, the scanning device and the treatment device need to avoid a region where the patient is located (as used herein, the region where the patient is located is referred to as a scanning and treatment space) in the process of rotation. Therefore, in addition to the scanning and treatment space, the larger the coverage area of the scanning rays and the treatment rays emitted by the scanning device and the treatment device, the smaller the imaging and treatment blind area of the scanning device and the treatment device. As used herein, the coverage area refers to an area that can be covered by scanning rays or treatment rays when the scanning device or the treatment device rotates around the patient to irradiate the patient from different angles. In some embodiments, the coverage area of the scanning rays and the treatment rays emitted by the scanning device and the treatment device may be related to irradiation angle ranges of the rays emitted by the scanning device and the treatment device, and rotation ranges of the scanning device and the treatment device. For example, the larger the irradiation angle ranges and the larger the rotation ranges of the scanning device and treatment device, the wider the coverage area of the scanning rays and the treatment rays emitted by the scanning device and the treatment device.

In some embodiments, a limiting device may be used to limit the rotation ranges of the scanning device and the treatment device to avoid the scanning and treatment space where the patient is located. However, arranging the limiting device may not only make an overall structure of the radiation imaging and treatment device more complex, but also increase the manufacturing cost. In addition, since the rotation ranges of the scanning device and the treatment device are limited, the coverage area of the rays emitted by the scanning device and the treatment device is reduced, which may reduce clinical work efficiency to a certain extent. Furthermore, the current radiation imaging and treatment device has a small irradiation angle, which leads to a small coverage area of the rays emitted by the scanning device and treatment device, resulting in a large imaging and treatment blind area of the scanning device and the treatment device, and making it unable to meet the requirements of complex clinical application scenarios.

Accordingly, the present disclosure provides a medical device to address the above-mentioned problems. The medical device may include a gantry, a first support member rotatably mounted on the gantry around a first rotation axis, and a treatment device. The treatment device may include a treatment ray emitter configured to emit treatment rays and a treatment ray receiver configured to receive the treatment rays. The treatment ray emitter and the treatment ray receiver may be rotatably mounted on the first support member around a second rotation axis synchronously. The first rotation axis may intersect with the second rotation axis.

In some embodiments, the medical device may further include a first scanning device and/or a second scanning device. The first scanning device may include a first scanning ray emitter configured to emit first scanning rays and a first scanning ray receiver configured to receive the first scanning rays. The first scanning ray emitter and the first scanning ray receiver may be rotationally mounted on the first support member around the second rotation axis synchronously. The second scanning device may include a second scanning ray emitter configured to emit second scanning rays and a second scanning ray receiver configured to receive the second scanning rays. The second scanning ray emitter and the second scanning ray receiver may be fixedly mounted on the first support member.

In the medical device and the medical system provided by the present disclosure, the treatment ray emitter and the treatment ray receiver may be mounted on the first support member, so that the first support member may drive the treatment device to rotate around the first rotation axis. Since the treatment ray emitter and the treatment ray receiver are rotatably mounted around the second rotation axis, and the first rotation axis intersects with the second rotation axis, the treatment device can rotate around two different rotation axes (the first rotation axis and the second rotation axis). Therefore, the embodiments of the present disclosure can increase the irradiation range of the treatment rays, and realize multi-angle and multi-directional adjustment of the irradiation angle of the treatment rays. Similarly, the irradiation range of the scanning rays can be increased, and multi-angle and multi-directional adjustment of the irradiation angle of the scanning rays can be realized. In addition, the scanning device and the treatment device can be prevented from interfering with the patient without the additional limiting device, which ensures the use safety of the medical device, and increases the irradiation range of the rays emitted by the scanning device and the treatment device, thereby reducing the manufacturing cost, and improving clinical work efficiency.

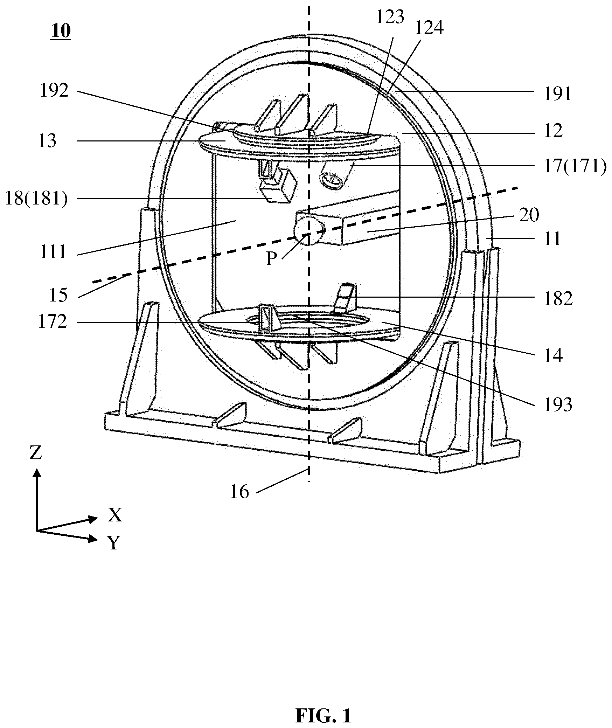

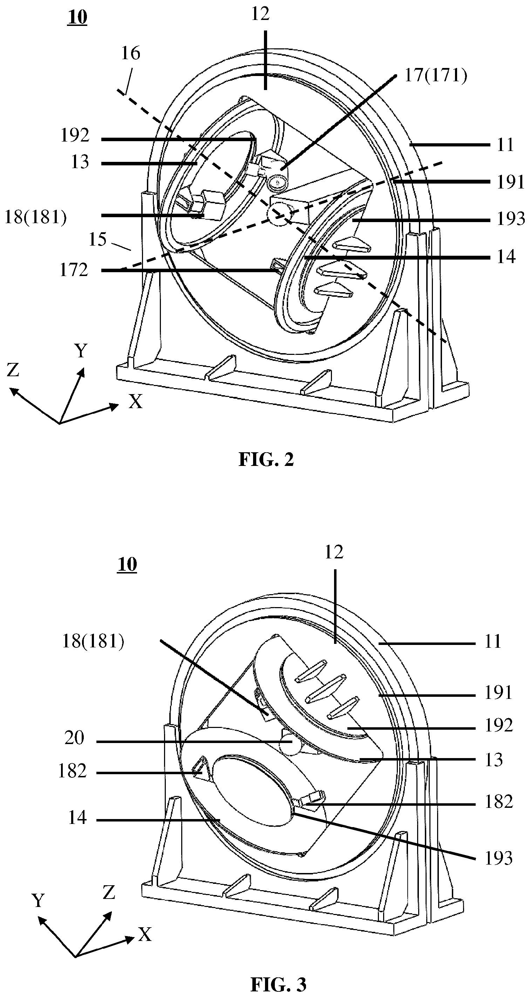

As illustrated in FIGs. 1-3, a medical device 10 may include a gantry 11, a first support member 12, a treatment device 17, and a first scanning device 18. The gantry 11 may be configured to support the first support member 12, the treatment device 17, and the first scanning device 18. The first support member 12 may be rotatably mounted on the gantry 11 around a first rotation axis 15.

The treatment device 17 includes a treatment ray emitter 171 and a treatment ray receiver 172. The treatment ray emitter 171 is configured to emit treatment rays. The treatment ray receiver 172 may be configured to receive the treatment rays emitted by the treatment ray emitter 171. The treatment ray emitter 171 and the treatment ray receiver 172 are rotatably mounted on the first support member 12 around a second rotation axis 16, and the treatment ray emitter 171 and the treatment ray receiver 172 rotate synchronously. The first rotation axis 15 intersects with the second rotation axis 16.

The first scanning device 18 may include a first scanning ray emitter 181 and a first scanning ray receiver 182. The first scanning ray emitter 181 may be configured to emit first scanning rays. The first scanning ray receiver 182 may be configured to receive the first scanning rays emitted by the first scanning ray emitter 181. The first scanning ray emitter 181 and the first scanning ray receiver 182 may be rotatably mounted on the first support member 12 around the second rotation axis 16, and the first scanning ray emitter 181 and the first scanning ray receiver 182 may rotate synchronously. The treatment device 17 and the first scanning device 18 are isocentric.

In some embodiments, an emission period of the first scanning rays may at least partially coincide with an emission period of the treatment rays. For example, a middle period of the emission period of the first scanning ray may coincide with a middle period of the emission period of the treatment rays. As another example, an end period of the emission period of the first scanning rays may coincide with an initial period of the emission period of the treatment rays. During a coincided period, a region to be treated may be imaged and treated simultaneously. In some embodiments, the emission period of the first scanning rays may not coincide with the emission period of the treatment rays. For example, the first scanning rays may be emitted before radiotherapy.

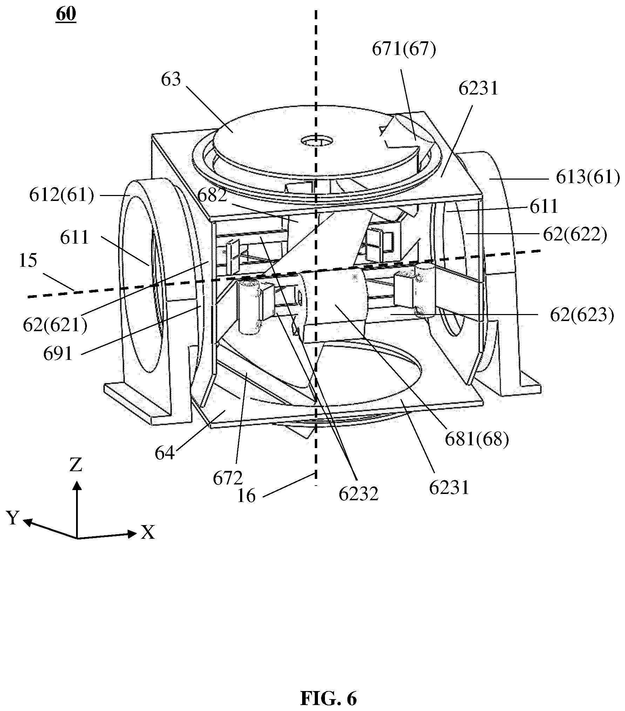

In some embodiments, the medical device 10 may further comprise a second scanning device. The second scanning device may be fixedly mounted on the first support member 12. More descriptions regarding the second scanning device may be found in FIG. 6, FIG. 7, and FIG. 9 and related descriptions thereof.

The first support member 12 being rotatably mounted on the gantry 11 around the first rotation axis 15 refers to that the first support member 12 may be mounted on the gantry 11 and may rotate around the first rotation axis 15 relative to the gantry 11. Synchronous rotation in the present disclosure refers to that two objects rotate around the same rotation axis in the same direction at the same speed. The treatment device 17 and the first scanning device 18 being isocentric refers to that the isocenter of the treatment device 17 coincides with the isocenter of the first scanning device 18. The isocenter of the treatment device 17 refers to a mechanical isocenter of the treatment device 17, i.e., an intersection point of treatment rays emitted by the treatment ray emitter 171 during a rotation process of the treatment ray emitter 171. Similarly, the isocenter of the first scanning device 18 refers to a mechanical isocenter of the first scanning device 18, i.e., an intersection point of first scanning rays emitted by the first scanning ray emitter 181 during a rotation process of the first scanning ray emitter 181. In some embodiments, the isocenter of the first scanning device 18 and the isocenter of the treatment device 17 are also referred to as an isocenter of the medical device 10.

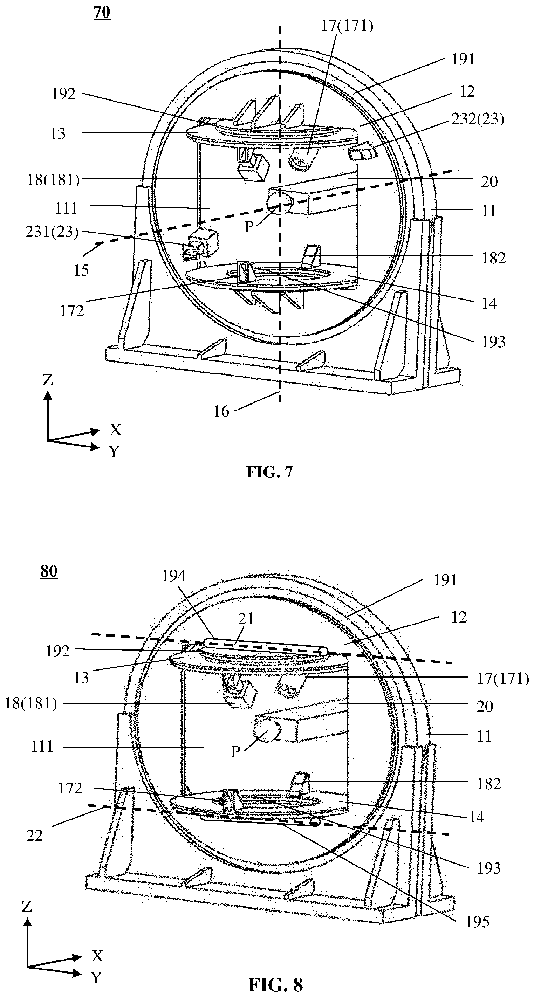

The first rotation axis 15 may intersect with the second rotation axis 16. That is, the first rotation axis 15 may not coincide with or parallel to the second rotation axis 16. In some embodiments, since the first rotation axis 15 intersects with the second rotation axis 16, the first rotation axis 15 may be perpendicular to or not be perpendicular to the second rotation axis 16. An intersection point of the first rotation axis 15 and the second rotation axis 16 may be the isocenter of the medical device 10, i.e., the isocenter of the treatment device 17 and the isocenter of the first scanning device 18. In some embodiments, an angle between the first rotation axis 15 and the second rotation axis 16 may be within a range of 0-90 degrees. The angle between the first rotation axis 15 and the second rotation axis 16 refers to a smaller pair of vertically opposite angles of angles between the first rotation axis 15 and the second rotation axis 16. In some embodiments, the angle between the first rotation axis 15 and the second rotation axis 16 may be within a range of 45-90 degrees. Merely by way of example, the first rotation axis 15 may be perpendicular to the second rotation axis 16, the first rotation axis 15 may be parallel to the X-axis direction as shown in FIG. 1, and the second rotation axis 16 may be parallel to the Z-axis direction as shown in FIG. 1. As another example, the angle between the first rotation axis 15 and the second rotation axis 16 may be 60 degrees. In some embodiments, the angle between the first rotation axis 15 and the second rotation axis 16 may be determined based on actual needs (e.g., a treatment range). In some embodiments, the angle between the first rotation axis 15 and the second rotation axis 16 may be fixed and determined when the medical device 10 is assembled. Alternatively, the angle between the first rotation axis 15 and the second rotation axis 16 may be dynamically adjusted. For example, the angle between the first rotation axis 15 and the second rotation axis 16 may be dynamically adjusted by the embodiments in FIG. 8. More descriptions may be found in the related descriptions of FIG. 8.

In some embodiments, a distance between the treatment ray emitter 171 during the rotation process and the lesion site may remain constant, thereby further accurately controlling a dose of the treatment rays. In addition, the treatment device 17 and the first scanning device 18 may be prevented from interfering with each other during the rotation process, effectively improving the use safety of the medical device 10.

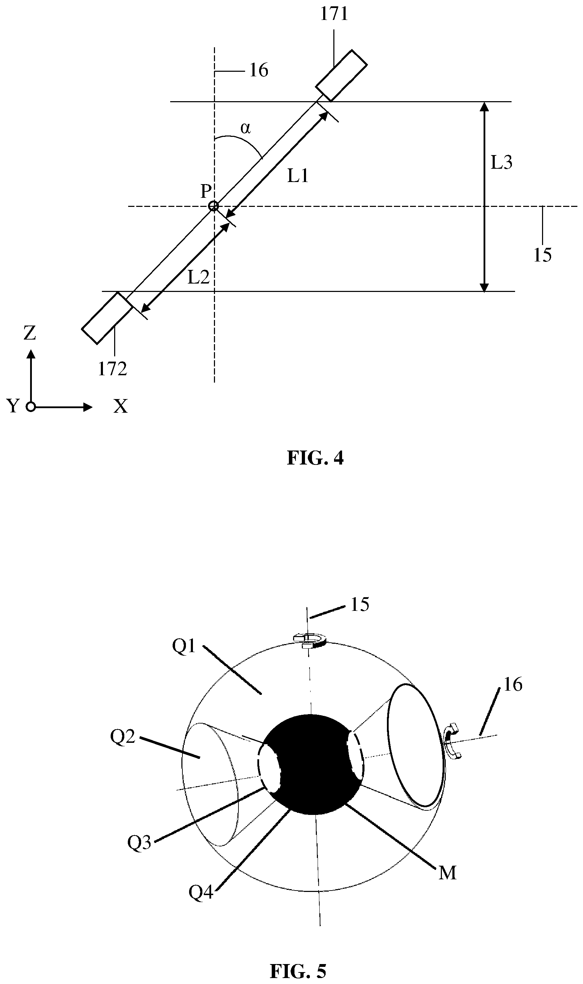

In some embodiments, a distance from the treatment ray emitter 171 to the isocenter of the treatment device 17 is referred to as a first isocenter distance, and a distance from the treatment ray receiver 172 to the isocenter of the treatment device 17 is referred to as a second isocenter distance. A sum of the first isocenter distance and the second isocenter distance may be greater than a size of the scanning and treatment space. For convenience of description, FIG. 4 provides a schematic diagram illustrating an exemplary irradiation angle of a treatment device. The first isocenter distance is denoted as L1. The second isocenter distance is denoted as L2. The isocenter of the treatment device 17 is denoted as P. The scanning and treatment space refers to a region that is inaccessible during the rotation process of the first scanning device 18 and the treatment device 17 and configured to accommodate the patient. The size of the scanning and treatment space is denoted as L3. In some embodiments, the sum of the first isocenter distance L1 and the second isocenter distance L2 may be greater than the size L3 of the scanning and treatment space, so as to prevent the treatment device 17 and the first scanning device 18 from colliding with the patient during the rotation process. In some embodiments, the first isocenter distance L1 and the second isocenter distance L2 may be the same or different. In some embodiments, the distance (i.e., the first isocenter distance) between the treatment ray emitter 171 and the isocenter of the treatment device 17 may remain constant during the rotation process, and/or the distance between the first scanning ray emitter 181 and the isocenter of the first scanning device 18 may remain constant during the rotation process, so that an irradiation dose of the first scanning rays and/or the treatment rays can be more accurately controlled to improve the accuracy of scanning and treatment, and the treatment device 17 and the first scanning device 18 can be prevented from interfering with each other during the rotation process. For example, a distance between the treatment ray emitter 171 and an isocenter of the medical device 10 (i.e., the isocenter of the treatment device 17) during the rotation process and a distance between the first scanning ray emitter 181 and the isocenter of the medical device 10 (i.e., the isocenter of the first scanning device 18) during the rotation process may remain constant.

Since the treatment ray emitter 171 and the treatment ray receiver 172 are mounted on the first support member 12, the treatment device 17 may be driven to rotate around the first rotation axis 15 through the first support member 12. Since the treatment ray emitter 171 and the treatment ray receiver 172 are rotatably mounted around the second rotation axis 16, the treatment ray emitter 171 and the treatment ray receiver 172 may rotate around the second rotation axis 16, and the first rotation axis 15 may intersect with the second rotation axis 16. That is to say, the treatment device 17 may rotate around two different rotation axes (the first rotation axis 15 and the second rotation axis 16), thereby increasing the irradiation range of the treatment rays, and realizing multi-angle and multi-directional adjustment of the irradiation range of the treatment rays.

The irradiation angle refers to an angle between irradiation rays and a straight line that is parallel to the second rotation axis and passes through an isocenter point. As illustrated in FIG. 4, the patient needs to be located in a middle region of the scanning and treatment space, the lesion site may coincide with the point P (isocenter), and the irradiation angle is denoted as α in FIG. 4. In some embodiments, the distance L1 from a treatment ray source (i.e., the treatment ray emitter 171) to the isocenter point may be determined based on the irradiation angle α, a structural size of the treatment ray emitter 171, and a reserved size L3 of the scanning and treatment space (i.e., a treatment aperture). For example, when the structural size of the treatment ray emitter 171 and the irradiation angle α remain constant, the closer the treatment ray emitter 171 is to an edge of the scanning and treatment space, the smaller the distance L1 from the treatment ray emitter 171 of the treatment device 17 to the isocenter point. The larger the structural size of the treatment ray emitter 171, the larger the space occupied by the treatment ray emitter 171, and the larger the distance from the treatment ray emitter 171 to the edge of the scanning and treatment space, which in turn increases the distance L1 from the treatment ray emitter 171of the treatment device 17 to the isocenter point. Therefore, when the irradiation angle α, the structural size of the treatment ray emitter 171, and the reserved size L3 of the scanning and treatment space are determined, a minimum value of the distance L1 from the treatment ray emitter 171 of the treatment device 17 to the isocenter point may be determined. In this way, the treatment ray emitter 171 can be mounted based on the minimum value of the distance L1 to avoid collision with the patient.

The irradiation range of rays refers to a region covered by the rays when the rays are directed at a three-dimensional target region (or referred to as the lesion site). FIG. 5 is a schematic diagram illustrating an exemplary irradiation model of treatment rays emitted by the treatment device 17. In the embodiments of FIG. 5, when the treatment device 17 rotates around the first rotation axis 15 and the second rotation axis 16, the treatment device 17 may irradiate a three-dimensional target region M at different irradiation angles. A region corresponding to Q1 represents an irradiation region that can be covered by treatment rays in space (i.e., the coverage area of the treatment rays in space), and a region corresponding to Q2 represents a non-irradiation region that can not be covered by the treatment rays. A region corresponding to Q4 represents a region on the three-dimensional target region M that can be covered by the treatment rays (i.e., the coverage area of the treatment rays on the three-dimensional target region M). A region corresponding to Q3 represents a region on the three-dimensional target area M that cannot be covered by the treatment rays. It can be seen from FIG. 5 that the embodiments of the present disclosure ensure that an area of the region covered by the treatment rays is large, and only a small part of the region is the non-irradiation region of the treatment rays, which indicates that the irradiation regions of the first scanning rays and the treatment rays emitted by the first scanning device 18 and the treatment device 17, respectively, in the embodiments of the present disclosure are relatively large.

Similarly, since the first scanning ray emitter 181 and the first scanning ray receiver 182 are disposed on the first support member 12, the first scanning device 18 may be driven to rotate around the first rotation axis 15 through the first support member 12. When the first scanning ray emitter 181 and the first scanning ray receiver 182 rotate around the second rotation axis 16 synchronously, it may be equivalent to that the first scanning device 18 rotates around two rotation axes. That is to say, the first scanning device 18 may rotate around two different rotation axes, which may increase the irradiation range of the first scanning rays, thereby realizing multi-angle and multi-directional adjustment of the irradiation angle of the first scanning rays.

In addition, since the first scanning device 18 and the treatment device 17 are both mounted on the first support member 12, the first scanning device 18 and the treatment device 17 may not interfere with each other during the rotation process, which may effectively improve the use safety of the medical device 10. Since the isocenter of the treatment device 17 always coincides with the isocenter of the first scanning device 18, no matter what position the treatment device 17 and the first scanning device 18 rotate to, scanning, imaging, and treatment can be performed on the lesion site if the patient 20 is in a same position, so that a relative positioning error in scanning, imaging and treatment can be avoided, thereby effectively improving the accuracy of radiotherapy.

In some embodiments, the treatment ray emitter 171 may include a linear accelerator, a cyclotron, a synchrotron, or the like. In some embodiments, the treatment rays may include relatively high energy beams (e.g., megavolt (MV) beams). In some embodiments, the first scanning ray emitter 181 may include an x-ray tube, the linear accelerator, or the like. In some embodiments, the first scanning rays may include relatively low energy beams (e.g., kilovolt (kV) beams). In some embodiments, the first scanning rays may include x-rays, γ-rays, α-rays, ultraviolet rays, radio frequency (RF), radar, laser, neutrons, protons, or the like, or any combinations thereof. In some embodiments, the treatment ray receiver 172 and the first scanning ray receiver 182 may include a flat panel detector or a curved surface detector.

In some embodiments, referring to FIGs. 1-3, the medical device 10 further includes a second support member 13 and a third support member 14. The second support member 13 and the third support member 14 are spaced apart from each other along an extension direction of the second rotation axis 16. The second support member 13 and the third support member 14 may be rotatably mounted on the first support member 12 around the second rotation axis 16 synchronously. The treatment ray emitter 171 may be disposed on one of the second support member 13 and the third support member 14, and the treatment ray receiver 172 may be disposed on the other of the second support member 13 and the third support member 14. The treatment ray receiver 172 may be disposed on a path of the treatment rays emitted by the treatment ray emitter 171, so that the treatment ray receiver 172 may receive the treatment rays emitted by the treatment ray emitter 171. Merely by way of example, the treatment ray emitter 171 may be disposed on the second support member 13, the treatment ray receiver 172 may be disposed on the third support member 14, and the treatment rays emitted by the treatment ray emitter 171 may be irradiated to the treatment ray receiver 172. Since the second support member 13 and the third support member 14 are mounted on the first support member 12 and rotate around the second rotation axis 16 synchronously, the second support member 13 and the third support member 14 may respectively drive the treatment ray emitter 171 and the treatment ray receiver 172 to rotate around the second rotation axis 16 synchronously to control the irradiation angle of the treatment rays.

In some embodiments, the first scanning ray emitter 181 may be disposed on one of the second support member 13 and the third support member 14, and the first scanning ray receiver 182 may be disposed on the other of the second support member 13 and the third support member 14. The first scanning ray receiver 182 may be disposed on a path of the first scanning rays emitted by the first scanning ray emitter 181, so that the first scanning ray receiver 182 may receive the first scanning rays emitted by the first scanning ray emitter 181. For example, in the embodiments of FIG. 1, the first scanning ray emitter 181 may be disposed on the second support member 13, the first scanning ray receiver 182 may be disposed on the third support member 14, and the first scanning rays emitted by the first scanning ray emitter 181 may be irradiated to the first scanning ray receiver 182.

In some embodiments, the first scanning ray emitter 181 and the treatment ray emitter 171 may both be disposed on one of the second support member 13 and the third support member 14, and the first scanning ray receiver 182 and the treatment ray receiver 172 may both be disposed on the other of the second support member 13 and the third support member 14, as long as the isocenter of the first scanning device 18 coincides with the isocenter of the treatment device 17. Merely by way of example, as illustrated in FIG. 1, the first scanning ray emitter 181 and the treatment ray emitter 171 may both be disposed on the second support member 13, and the first scanning ray receiver 182 and the treatment ray receiver 172 may both be disposed on the third support member 14. As another example, the first scanning ray emitter 181 and the treatment ray emitter 171 may both be disposed on the third support member 14, and the first scanning ray receiver 182 and the treatment ray receiver 172 may both be disposed on the second support member 13.

In some embodiments, the first scanning ray emitter 181 and the treatment ray emitter 171 may be disposed on different support members. For example, the first scanning ray emitter 181 may be disposed on the third support member 14, the first scanning ray receiver 182 may be disposed on the second support member 13, the treatment ray emitter 171 may be disposed on the second support member 13, and the treatment ray receiver 172 may be disposed on the third support member 14. In some embodiments, referring to FIGs. 1-4, when the first scanning ray emitter 181 and the treatment ray emitter 171 are both disposed on the second support member 13 or the third support member 14, the first scanning ray emitter 181 and the treatment ray emitter 171 may be arranged symmetrically with respect to the second rotation axis 16. Alternatively, the first scanning ray emitter 181 and the treatment ray emitter 171 may be arranged asymmetrically with respect to the second rotation axis 16. A distance from the first scanning ray emitter 181 to the second rotation axis 16 and a distance from the treatment ray emitter 171 to the second rotation axis may be set based on use requirements of the medical device, respectively. Similarly, when the first scanning ray receiver 182 and the treatment ray receiver 172 are both disposed on the second support member 13 or the third support member 14, the first scanning ray receiver 182 and the treatment ray receiver 172 may be arranged symmetrically with respect to the second rotating axis 16. Alternatively, the first scanning ray receiver 182 and the treatment ray receiver 172 may be arranged asymmetrically with respect to the second rotation axis 16. A distance from the first scanning ray receiver 182 to the second rotation axis 16 and a distance from the treatment ray receiver 172 to the second rotation axis 16 may be set based on the use requirements of the medical device, respectively. In some embodiments, by disposing the first scanning ray emitter 181 and the first scanning ray receiver 182 on the second support member 13 and the third support member 14, respectively, the first scanning ray emitter 181 and the first scanning ray receiver 182 rotate around the second rotation axis 16 synchronously, and the first scanning device 18 and the treatment device 17 can move synchronously.

In some embodiments, the first support member 12 may be rotationally connected with the gantry 11 by means including a gear mechanism, a cam mechanism, a conveyor belt mechanism, a worm gear mechanism, and a rotary bearing mechanism. Merely by way of example, as illustrated in FIGs. 1-4, the medical device 10 may include a first bearing 191. An outer ring (i.e., a fixed portion) of the first bearing 191 may be connected with the gantry 11, and an inner ring (i.e., a rotation portion) of the first bearing 191 may be connected with the first support member 12. An axial direction of the first bearing 191 may coincide with the first rotation axis 15, so that the first support member 12 may rotate around the first rotation axis 15 relative to the gantry 11.

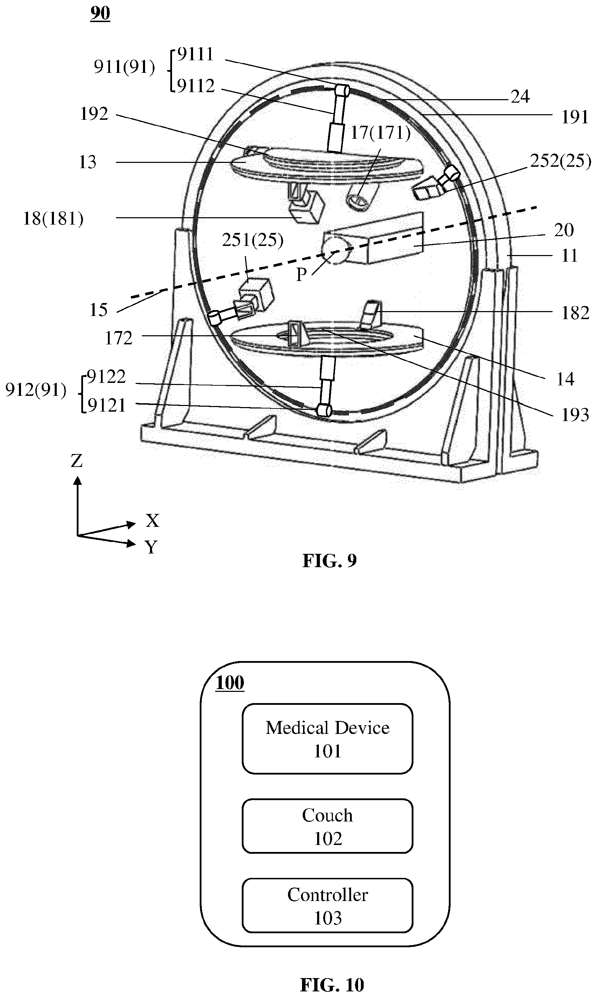

In some embodiments, the medical device 10 may include a slide rail disposed along a circumferential direction of the gantry 11. The first support member 12 may include a first sliding device and a second sliding device. The first sliding device may be configured to slidably mount the treatment ray emitter 171 in the slide rail. The second sliding device may be configured to slidably mount the treatment ray receiver 172 in the slide rail. More descriptions regarding the slide rail and the sliding devices may be found in FIG. 9 and related descriptions thereof.

In some embodiments, the second support member 13 may be connected with the first support member 12 through a second bearing 192. The third support member 14 may be connected with the first support member 12 through a third bearing 193. A rotatable connection of the second support member 13 with the first support member 12 and a rotatable connection of the third support member 14 with the first support member 12 may be the same as or similar to a rotatable connection of the first support member 12 with the gantry 11, which are not repeated here.

In some embodiments, the second support member 13 may be rotatably mounted on the first support member 12 around a third rotation axis. The third support member 14 may be rotatably mounted on the first support member 12 around a fourth rotation axis. More descriptions regarding mounting modes of the second support member 13 and the third support member 14 may be found in FIG. 8 and related descriptions thereof.

In some embodiments, the first support member 12 may include an annular structure. The gantry 11 may include an accommodating hole 111. The annular structure may be mounted within the accommodating hole 111. The second support member 13 and the third support member 14 may be connected with an inner ring 123 of the first support member 12. The accommodating hole 111 of the gantry 11 may be configured to accommodate a couch, the patient 20, the first support member 12, etc. The annular structure refers to a structure with a hollow interior. The annular structure may include an outer ring 124 and the inner ring 123. The outer ring 124 may be connected with an inner wall of the accommodating hole 111 (e.g., connected with the accommodating hole 111 through the first bearing 191). The inner ring 123 may be connected with the second support member 13 and the third support member 14 (e.g., connected with the second support member 13 and the third support member 14 through the second bearing 192 and the third bearing 193, respectively). In some embodiments, a shape of the accommodating hole 111 may include regular or irregular shapes such as a circle, an ellipse, a square, etc. A shape of the outer ring 124 of the annular structure may match the shape of the accommodating hole 111. For example, in the embodiments of FIG. 1, the shape of the accommodating hole 111 and the shape of the outer ring 124 of the annular structure may be the circle, respectively. Similarly, in some embodiments, the shape of the inner ring 123 of the annular structure may include the regular or irregular shapes such as the circle, the ellipse, the square, etc. For example, in the embodiments of FIG. 1, the shape of the inner ring 123 of the annular structure may be the square. In some embodiments, by arranging the first support member 12 in the form of the annular structure, the outer ring 124 of the first support member 12 may be connected with the gantry 11, and the inner ring 123 of the first support member 12 may be connected with the second support member 13 or the third support member 14, thereby making an overall structural layout of the medical device 10 more compact, and occupy less space.

In some embodiments, referring to FIGs. 1-3, the medical device 10 may include a driving component (not shown in the figures). The driving component may be configured to drive the support members to move. In some embodiments, the driving component may include a first driving member, a second driving member, and a third driving member. The first driving member may be connected with the first support member 12 to drive the first support member 12 to move. The second driving member may be connected with the second support member 13 to drive the second support member 13 to move. The third driving member may be connected with the third support member 14 to drive the third support member 14 to move. In some embodiments, the first driving member, the second driving member, and the third driving member may include a power mechanism and a transmission mechanism, respectively. An output member of the power mechanism may be connected with the transmission mechanism to transmit power to the corresponding support member through the transmission mechanism to drive the corresponding support member to move. In some embodiments, the transmission mechanism may include a gear transmission mechanism, a cam transmission mechanism, a conveyor belt transmission mechanism, a worm gear transmission mechanism, or the like. In some embodiments, the power mechanism may include an electric motor, a hydraulic cylinder, a pneumatic cylinder, or the like.

In some embodiments, the driving component may include a synchronous movement mechanism (not shown in the figure). The synchronous movement mechanism may be connected with the second driving member and the third driving member, so that the second driving member and the second driving member may move synchronously. Merely by way of example, the second support member 13 and the third support member 14 may be powered by the same power mechanism, and the power may be transmitted to the second support member 13 and the third support member 14 through the gear transmission mechanism. The synchronous movement mechanism may include a synchronous belt transmission mechanism. A driving gear that drives the second support member 13 and a driving gear that drives the third support member 14 may be connected with two ends of the synchronous belt transmission mechanism, respectively, to realize the synchronous movement of the second support member 13 and the third support member 14. As another example, the medical device 10 may include a controller (e.g., a controller 103 of the medical system 100). The controller may be in communicating connection with the second driving member and the third driving member. The controller may control output shafts of the second driving member and the third driving member to rotate in a same direction at a same speed, thereby realizing the synchronous movement.

FIG. 6 is a schematic diagram illustrating an exemplary medical device 60 according to some embodiments of the present disclosure.

As illustrated in FIG. 6, the medical device 60 may include a treatment device 67, a second scanning device 68, and a first support member 62. The treatment device 67 may include a treatment ray emitter 671 and a treatment ray receiver 672. The treatment ray emitter 671 and a treatment ray receiver 672 may be rotatably mounted on the first support member 62 around the second rotation axis 16 synchronously. The treatment device 67 may be similar to the treatment device 17, the descriptions of which are not repeated here.

The second scanning device 68 may include a second scanning ray emitter 681 configured to emit second scanning rays and a second scanning ray receiver 682 configured to receive the second scanning rays. The second scanning ray emitter 681 and the second scanning ray receiver 682 may be fixedly mounted on the first support member 62. The second scanning ray emitter 681 and the second scanning ray receiver 682 may be disposed on two sides of a plane (i.e., a Z-X plane) defined by the first rotation axis 15 and the second rotation axis 16, respectively. Since the first rotation axis 15 intersects with the second rotation axis 16, the first rotation axis 15 and the second rotation axis 16 may define a unique plane.

In some embodiments, a connection line between the second scanning ray emitter 681 (where an emitter head of the second scanning ray emitter 681 is located) and the second scanning ray receiver 682 (where a receiver head of the second scanning ray receiver 682 is located) may have an intersection point with the first rotation axis 15. The intersection point may be an isocenter point of the second scanning device 68. When the first support member 62 rotates around the first rotation axis 15, the second scanning rays emitted by the second scanning ray emitter 681 may always be received by the second scanning ray receiver 682.

In some embodiments, the second scanning ray emitter 681 and the second scanning ray receiver 682 may be arranged symmetrically with respect to the plane defined by the first rotation axis 15 and the second rotation axis 16. For example, a distance from the second scanning ray emitter 681 to the isocenter point of the second scanning device 68 may be equal to a distance from the second scanning ray receiver 682 to the isocenter point of the second scanning device 68. In some embodiments, the second scanning ray emitter 681 and the second scanning ray receiver 682 may arranged asymmetrically with respect to the plane defined by the first rotation axis 15 and the second rotation axis 16. The distance from the second scanning ray emitter 681 to the isocenter point of the second scanning device 68 and the distance from the second scanning ray receiver 682 to the isocenter point of the second scanning device 68 may be set based on use requirements of the medical device 60.

In some embodiments, in order to reduce a transmission distance of the second scanning rays and correspondingly reduce an energy loss during the transmission of the second scanning rays, the connection line between the second scanning ray emitter 681 and the second scanning ray receiver 682 may be perpendicular or approximately perpendicular to the plane defined by the first rotation axis 15 and the second rotation axis 16.

In some embodiments, an emission period of the second scanning rays may not coincide with an emission period of treatment rays. For example, the second scanning device 68 may first emit the second scanning rays, and then the treatment device may emit the treatment rays. As another example, the treatment device may first emit the treatment rays, and then the second scanning device may emit the second scanning rays. In some embodiments, the emission period of the second scanning rays may at least partially coincide with the emission period of the treatment rays. A region to be treated may be treated and imaged simultaneously during a coincided period.

In some embodiments, as illustrated in FIG. 6, a gantry 61 may include a first gantry component 612 and a second gantry component 613. The first gantry component 612 and the second gantry component 613 may be spaced apart from each other along an extension direction of the first rotation axis 15. Two ends of the first support member 62 along the extension direction of the first rotating axis 15 may be rotationally connected with the first gantry component 612 and the second gantry component 613, respectively. The second scanning ray emitter 681 and the second scanning ray receiver 682 may be disposed on the first support member 62 between the first gantry component 612 and the second gantry component 613 to rotate around the first rotation axis 15 along with the first support member 62.

In some embodiments, by providing the first gantry component 612 and the second gantry component 613 to support the first support member 62, the stability of the first support member 62 can be improved. In addition, the better the imaging performance of the second scanning device 68, the heavier the second scanning device 68. By providing the first gantry component 612 and the second gantry component 613 for support, the support stability can be enhanced, and the second scanning device with better performance and heavier weight can also operate stably.

In some embodiments, as illustrated in FIG. 6, the first support member 62 may include a first support end portion 621, a second support end portion 622, and a connecting portion 623. The connecting portion 623 may be connected between the first support end portion 621 and the second support end portion 622. The first support end portion 621 may be rotatably connected with the first gantry component 612. The second support end portion 622 may be rotatably connected with the second gantry component 613. The second support member 63 and the third support member 64 may be rotatably mounted on the connecting portion 623 around the second rotation axis 16. The second scanning ray emitter 681 and the second scanning ray receiver 682 may be connected to the connecting portion 623.

In the embodiments, the second scanning ray emitter 681 and the second scanning ray receiver 682 may rotate around the first rotation axis 5 through the first support end portion 621 and the second support end portion 622. In addition, since the second support member 63 and the third support member 64 are disposed on the connecting portion 623, the treatment ray emitter 671 and the treatment ray receiver 672 may rotate around the second rotation axis 16 through the second support member 63 and the third support member 623. That is, the second scanning device 68 may rotate around the first rotation axis 15, and the treatment device 67 may rotate around the first rotation axis 15 and the second rotation axis 16.

In some embodiments, the first support member 62 may have a frame structure, and each of the first support end portion 621 and the second support end portion 622 may have a square, a circle, an ellipse, or other regular or irregular plate-shaped or block-shaped structure. The connecting portion 623 may include at least two rods, plates or blocks configured to connect the first support end portion 621 and the second support end portion 622. In some embodiments, the first support member 62 may have a cylindrical structure. For example, the first support member 62 may be a cylinder or a square cylinder.

In some embodiments, the first gantry component 612 and the second gantry component 613 may include an accommodating hole 611, respectively. The first support end portion 621 and the second support end portion 622 may include an annular structure, respectively. The annular structure may be mounted within the accommodation hole 611 and rotationally connected with the accommodating hole 611. In some embodiments, a first bearing 691 may be provided between the accommodating hole 611 and the annular structure, so that the accommodating hole 611 may be rotatably connected with the annular structure. Specifically, the first gantry component 612 and the second gantry component 613 may be connected and fixed with an outer ring of the first bearing 691 through the accommodating hole 611, respectively. The first support end portion 621 and the second support end portion 622 may be connected and fixed with an inner ring of the first bearing 691 through the annular structure, respectively. In some embodiments, the accommodating hole 611 may match a shape of the outer ring of the first bearing 691. For example, the accommodating hole 611 may have a regular or irregular shape such as a circle or a square. In some embodiments, the accommodating holes 611 may have a circular hole structure. In some embodiments, the annular structure may match a shape of the inner ring of the first bearing 691.

In some embodiments, as illustrated in FIG. 6, when the connecting portion 623 includes two rods 6232, the two rods 6232 may be disposed on two sides of a plane defined by the first rotation axis 15 and the second rotation axis 16, respectively. Two ends of the two rods 6232 may be connected with the first gantry component 612 and the second gantry component 613, respectively. The second scanning ray emitter 681 and the second scanning ray receiver 682 may be connected on the two rods 6232, respectively. In some embodiments, the second scanning ray emitter 681 and the second scanning ray receiver 682 may be fixedly connected or detachably connected with the connecting portion 623. A fixed connection may include bonding, welding, etc. A detachable connection may include a snap connection, a threaded connection, etc.

In some embodiments, as illustrated in FIG. 6, the second support member 63 may be connected with the connecting portion 623 of the first support member 62 through a second bearing (not shown in the figure), and the third support member 64 may be connected with the connecting portion 623 through a third bearing (not shown in the figure). In some embodiments, the connection portion 623 may include two plates 6231. Two ends of the two plates 6231 along an extension direction of the first rotation axis 15 may be connected with the first gantry component 612 and the second gantry component 612, respectively. The treatment device 67 may be disposed on the two plates 6231. Merely by way of example, in the embodiments of FIG. 6, the connecting portion 623 may include the two plates 6231 and the two rods 6232. Each of the two plates 6231 may have a rectangular plate structure. The two plates 6231 and the two rods 6232 may be spaced apart from each other around a circumferential direction of the first rotation axis 15. Two ends of each of the two plats 6231 and each of the two rods 6232 along the extension direction of the first rotation axis 15 may be connected with the first support end portion 621 and the second support end portion 622, respectively. The second support member 63 and the third support member 64 may be rotatably disposed on the two plates 6231 through the second bearing (not shown in the figure) and the third bearing (not shown in the figure), respectively, so that the second support member 63 and the third support member 64 may rotate around the second rotation axis 16 synchronously.

In some embodiments, the second support member 63 may be rotatable mounted on the upper plate 6231 around an axis parallel to the first rotation axis 15. For example, the installation manner of the second support member 63 may be similar to the second support member 13 as described in connection with FIG. 8. In some embodiments, the treatment ray emitter 671 may be rotatably mounted on the second support member 63 so that an irradiation angle of the treatment rays can be adjusted flexibly.

FIG. 7 is a schematic diagram illustrating an exemplary medical device 70 according to some embodiments of the present disclosure. The medical device 70 may be similar to the medical device 10, except that the medical device 70 includes both the first scanning device 18 and a second scanning device 23.

The first scanning device 18 may include the first scanning ray emitter 181 configured to emit first scanning rays and the first scanning ray receiver 182 configured to receive the first scanning rays. The first scanning ray emitter 181 and the first scanning ray receiver 182 may be rotatably mounted on the first support member 12 around the second rotation axis 16 simultaneously. The second scanning device 23 may include a second scanning ray emitter 231 configured to emit second scanning rays and a second scanning ray receiver 232 configured to receive the second scanning rays. The second scanning ray emitter 231 and the second scanning ray receiver 232 may be fixedly mounted on the first support member 12.

In some embodiments, the second scanning ray emitter 231 and the second scanning ray receiver 232 may be disposed on two sides of a plane defined by the first rotation axis 15 and the second rotation axis 16 (i.e., a Z-X plane). In some embodiments, the second scanning ray emitter 231 and the second scanning ray receiver 232 may be mounted at any position on the first support member 12 (e.g., an interior of an inner ring, a position between the inner ring and an outer ring, etc.), as long as the second scanning ray receiver 232 is located on a transmission path of the second scanning rays emitted by the second scanning ray emitter 231. In some embodiments, a connection line between the second scanning ray emitter 231 (where an emitter head of the second scanning ray emitter 231 is located) and the second scanning ray receiver 231 (where a receiver head of the second scanning ray receiver 231 is located) may have an intersection point with the first rotation axis 15. The intersection point may be an isocenter point of the second scanning device 23. When the first support member 12 rotates around the first rotation axis 15, the second scanning rays emitted by the second scanning ray emitter 231 may always be received by the second scanning ray receiver 232. In some embodiments, the second scanning ray emitter 231 and the second scanning ray receiver 232 may be arranged symmetrically or asymmetrically relative to the plane defined by the first rotation axis 15 and the second rotation axis 16. In some embodiments, a mounting mode of the second scanning device 23 may be similar to the mounting mode of the second scanning device 68 in FIG. 6, the descriptions of which are not repeated here.

In some embodiments, the first scanning device 18 and the second scanning device 23 may be configured to image a region to be treated before radiotherapy (i.e., before treatment rays are emitted), during radiotherapy (i.e., when the treatment rays are emitted), and/or after radiotherapy (i.e., after the treatment rays are emitted). In some embodiments, the first scanning device 18 may be configured to image the region to be treated during radiotherapy, thereby acquiring a treatment image. In this case, an emission period of the first scanning rays may at least partially coincide with an emission period of treatment rays. The treatment image acquired by the first scanning device 18 during radiotherapy may be used for monitoring (e.g., tracking movement) of the region to be treated, thereby realizing dynamic adjustment of radiotherapy, and improving treatment accuracy. Since the first scanning device 18 and the treatment device 17 are mounted on the second support member 13, the first scanning device 18 and the treatment device 17 may move synchronously, and the treatment image acquired by the first scanning device 18 during radiotherapy may better guide radiotherapy. In some embodiments, the second scanning device 23 may be configured to image the region to be treated before radiotherapy, thereby acquiring a planning image. In this case, the emission period of the second scanning rays may not coincide with the emission period of the treatment rays. The planning image acquired by the second scanning device 23 before radiotherapy may be used to formulate a treatment plan.

In some embodiments, the first scanning rays and the second scanning rays may be the same type or different types of beams. For example, the first scanning rays and the second scanning rays may be x-rays. A dose of the first scanning rays and a dose of the second scanning rays may be determined based on actual needs. The dose of the first scanning rays and the dose of the second scanning rays may be the same or different. In some embodiments, when the second scanning device 23 is used for imaging before treatment and the first scanning device 18 is used for imaging during treatment, the dose of the second scanning rays may be higher than the dose of the first scanning rays. It should be understood that the planning image needs a higher image quality to ensure the accuracy of the treatment plan; while a quality requirement for the treatment image may be relatively low. Therefore, the planning image may be acquired using the second scanning rays of a relatively high dose, and the treatment image may be acquired using the first scanning rays of a relatively low dose, thereby improving the accuracy of the treatment plan, and avoiding excessive radiation to the patient during treatment.

In some embodiments, the first scanning device 18 may include a digital radiographic (DR) device, and the second scanning device 23 may include a computed tomographic (CT) device.

FIG. 8 is a schematic diagram illustrating an exemplary medical device 80 according to some embodiments of the present disclosure. The medical device 80 may be similar to the medical device 10, except that the second support member 13 and the third support member 14 are mounted in different ways.

As illustrated in FIG. 8, the second support member 13 may be rotatably mounted on the first support member 12 around the third rotation axis, and the third rotation axis may be parallel to an extension direction of an intersection line between the second support member 13 and the first support member 12. The third support member 14 may be rotatably mounted on the first support member 12 around the fourth rotation axis. The fourth rotation axis may be parallel to an extension direction of an intersection line between the third support member 14 and the first support member 12. The third rotation axis may be parallel to the fourth rotation axis. Specifically, the dotted line 21 in FIG. 8 represents the intersection line between the second support member 13 and the first support member 12, the dotted line 22 represents the intersection line between the third support member 14 and the first support member 12, the third rotation axis coincides with the intersection line 21, the fourth rotation axis coincides with the intersection line 22, and the intersection line 21 is parallel to the intersection line 22.

The second support member 13 being rotatably mounted on the first support member 12 around the third rotation axis refers to that the second support member 13 may be mounted on the first support member 12 and may rotate around the third rotation axis relative to the first support member 12. The third support member 14 being rotatably mounted on the first support member 12 around the fourth rotation axis refers to that the third support member 14 may be mounted on the first support member 12 and may rotate around the fourth rotation axis relative to the first support member 12. In some embodiments, the second support member 13 and the third support member 14 may rotate synchronously around the third rotation axis and the fourth rotation axis, respectively. During a rotation process, the second support member 13 and the third support member 14 may always be opposite each other, and an isocenter of the treatment device 17 may coincide with an isocenter of the first scanning device 18. In some embodiments, the second support member 13 and the third support member 14 may rotate in a limited angle range to avoid collision with the gantry 11 or the patient and prevent the treatment rays and the first scanning rays from being blocked by the gantry 11.

In some embodiments, as mentioned above, the treatment ray emitter 171 and the first scanning ray emitter 181 may be disposed on one of the second support member 13 and the third support member 14, and the treatment ray receiver 172 and the first scanning ray receiver 182 may be disposed on the other of the second support member 13 and the third support member 14. For example, as illustrated in the embodiment of FIG. 8, the treatment ray emitter 171 and the first scanning ray emitter 181 may be disposed on the second support member 13, and the treatment ray receiver 172 and the first scanning ray receiver 182 may be disposed on the third support member 14. Therefore, the treatment ray emitter 171 and the first scanning ray emitter 181 may be driven to rotate around the third rotation axis through the second support member 13. The treatment ray receiver 172 and the first scanning ray receiver 182 may be driven to rotate around the fourth rotation axis through third support member 14.

In some embodiments, the second support member 13 may be connected with the first support member 12 through a fourth bearing 194, so that the second support member 13 may rotate around the third rotation axis. The third support member 14 may be connected with the first support member 12 through a fifth bearing 195, so that the third support member 14 may rotate around the fourth rotation axis. A rotatable connection mode of the second support member 13, the third support member 14, and the first support member 12 may be the same as or similar to the aforementioned rotatable connection mode of the first support member 12 and the gantry 11, which is not repeated here.

In some embodiments of the present disclosure, the second support member 13 and the third support member 14 may be rotatably mounted on the first support member 12 around the third rotation axis and the fourth rotation axis, respectively, so that the second support member 13 and the third support member 14 may rotate to drive the treatment device 17 and the first scanning device 18 to rotate around the third rotation axis and the fourth rotation axis, respectively, which can increase a rotation range of the treatment device 17 and the first scanning device 18, thereby enlarging an irradiation range of the treatment rays and the first scanning rays, and realizing multi-angle and multi-directional adjustment of an irradiation angle of the treatment rays and the first scanning rays. In addition, when the second support member 13 and the fourth support member 14 rotate to a different angle around the third rotation axis and the fourth rotation axis, a direction of the second rotation axis may change, so that an angle between the first rotation axis and the second rotation axis may be adjusted according to different needs.

In some embodiments, the treatment ray emitter 171 and/or the first scanning ray emitter 181 may be rotatably mounted on the second support member 13. Additionally or alternatively, the treatment ray receiver 172 and/or the first scanning ray receiver 182 may be rotatably mounted on the fourth support member 14. For example, the irritation angle of the treatment rays may be adjusted by rotating the treatment ray emitter 171, and the treatment ray receiver 172 may be adjusted to receive the treatment rays. In this way, the irradiation range of the treatment rays and the first scanning rays can be further enlarged.

FIG. 9 is a schematic diagram illustrating an exemplary medical device 90 according to some embodiments of the present disclosure. The medical device 90 may be similar to the medical device 10, except that a structure of a first support member configured to mount the treatment device 17 and the first scanning device 18 in the medical device 90 is different.

As illustrated in FIG. 9, the medical device 90 may include a slide rail 24 arranged along a circumferential direction of the gantry 11. An axial direction of the slide rail 24 may coincide with the first rotation axis 15. A first support member 91 may include a first sliding device 911 and a second sliding device 912. The first sliding device 911 may be configured to slidably mount the treatment ray emitter 171 in the slide rail 24, and the second sliding device 912 may be configured to slidably mount the treatment ray receiver 172 in the slide rail 24. The first sliding device 911 and the second sliding device 912 may be symmetrically arranged on two sides of the first rotation axis 15.

In some embodiments, the treatment ray emitter 171 may be directly or indirectly connected with the first sliding device 911. For example, as illustrated in FIG. 9, the treatment ray emitter 171 may be indirectly connected with the first sliding device 911 through the second support member 13. The first sliding device 911 may drive the second support member 13 to rotate around the first rotation axis 15 in the slide rail 24, thereby driving the treatment ray emitter 171 to rotate around the first rotation axis 15. As another embodiment, the treatment ray emitter 171 may be directly connected with the first sliding device 911, and the first sliding device 911 may rotate around the first rotation axis 15 in the slide rail 24, thereby driving the treatment ray emitter 171 to rotate around the first rotation axis 15. A connection mode of the second sliding device 912 and the treatment ray receiver 172 may be similar to a connection mode of the first sliding device 911 and the treatment ray emitter 171, which is not repeated here.

In some embodiments, the first sliding device 911 and the second sliding device 912 may rotate synchronously around the first rotation axis 15, thereby driving the treatment ray emitter 171 and the treatment ray receiver 172 to rotate synchronously around the first rotation axis 15, so that the treatment ray receiver 172 may receive treatment rays emitted by the treatment ray emitter 171.

In some embodiments, the first sliding device 911 and the second sliding device 912 may be directly or indirectly connected with the first scanning device 18. For example, the first sliding device 911 may be directly or indirectly connected with the first scanning ray emitter 181, and the second sliding device 912 may be directly or indirectly connected with the first scanning ray receiver 182. Merely by way of example, as illustrated in FIG. 9, the first scanning ray emitter 181 may be indirectly connected with the first sliding device 911 through the second support member 13. The first sliding device 911 may drive the second support member 13 to rotate around the first rotation axis 15 in the slide rail 24, thereby driving the first scanning ray emitter 181 to rotate around the first rotation axis 15.

In some embodiments, as illustrated in FIG. 9, the first sliding device 911 may include a first pulley 9111 mounted in the slide rail 24 and a first connecting rod 9112 configured to connect the first pulley 9111 and the treatment ray emitter 171. The second sliding device 912 may include a second pulley 9121 mounted in the slide rail 24 and a second connecting rod 9122 configured to connect the second pulley 9121 and the treatment ray receiver 172. The first pulley 9111 and the second pulley 9121 may slide in the slide rail 24. The first connecting rod 9112 and the second connecting rod 9122 may be directly or indirectly connected with the treatment ray emitter 171 and the treatment ray receiver 172, thereby driving the treatment ray emitter 171 and the treatment ray receiver 172 to rotate synchronously around the first rotation axis 15.

In some embodiments, a plurality of first connecting rods 9112 and a plurality of second connecting rods 9122 may be provided. For example, one first connecting rod 9112 and one second connecting rod may be provided, two first connecting rods 9112 and two second connecting rods may be provided, three first connecting rods 9112 and three second connecting rods may be provided, etc. Taking the first connecting rod 9112 as an example, as illustrated in FIG. 9, one first connecting rod 9112 may be provided. In some embodiments, two or more first connecting rods 9112 may be provided to improve a connection strength and a connection stability of the two or more first connecting rods 9112, thereby improving a mounting stability of the treatment device 17. In some embodiments, two first connecting rods 9112 may be provided to connect the treatment ray emitter 171 and the first scanning ray emitter 181, respectively, and two second connecting rods 9122 may be provided to connect the treatment ray receiver 172 and the first scanning ray emitter 181, respectively.

In some embodiments, a length of the first connecting rod 9112 and/or the second connecting rod 9122 may be fixed. In some embodiments, as illustrated in FIG. 9, the length of the first connecting rod 9112 may be adjusted to adjust a distance from the treatment ray emitter 171 to the gantry 11. The length of the second connecting rod 9122 may be adjusted to adjust a distance from the treatment ray receiver 172 to the gantry 11. The lengths of the first connecting rod 9112 and the second connecting rod 9122 may be adjusted through cylinder adjustment, knob adjustment, etc. In some embodiments, when the first sliding device 911 and the second sliding device 912 are connected with the first scanning device 18, the distance from the first scanning ray emitter 181 to the gantry 11 and the distance from the second scanning ray receiver 182 to the gantry 11 may be adjusted through the first connecting rod 9112 and the second connecting rod 9122. In some embodiments, when the lengths of the first connecting rod 9112 and the second connecting rod 9122 are adjusted, an isocenter of the treatment device 17 may coincide with an isocenter of the first scanning device 18.

A distance from the treatment device 17 and/or the first scanning device 18 to the gantry 11 or the patient 20 may be adjusted by adjusting the lengths of the first connecting rod 9112 and the second connecting rod 9122, so that radiotherapy for different types of patients (e.g., children or adults) or different lesion sites (e.g., a head or a chest) can be realized, thereby meeting different treatment needs.

In some embodiments, as illustrated in FIG. 9, the medical device 90 may further include a third scanning device 25. The third scanning device 25 may include a third scanning ray emitter 251 and a third scanning ray receiver 252. The third scanning ray emitter 251 and the third scanning ray receiver 252 may be slidably mounted in the slide rail 24 through sliding devices, respectively. The sliding devices may slide in the slide rail 24 to drive the third scanning ray emitter 251 and the third scanning ray receiver 252 to rotate around the first rotation axis 15. In some embodiments, the third scanning device 25 may be configured to image a region to be treated before radiotherapy (i.e., before treatment rays are emitted), during radiotherapy (i.e., when the treatment rays are emitted), and/or after radiotherapy (i.e., after the treatment rays are emitted). In some embodiments, the function of the third scanning device 25 may be similar to that of the second scanning device 23.

In some embodiments of the present disclosure, the treatment device and the scanning devices may be mounted using the slide rail and the sliding devices, which can realize multidimensional rotation of the treatment device and the scanning devices, increase an irradiation range of the treatment rays and the scanning rays, and realize multi-angle and multi-directional adjustment of irradiation angles of the treatment rays and the scanning rays. In addition, the treatment device and the scanning devices may be flexibly mounted and removed through the sliding devices. Furthermore, any count of scanning devices may be mounted through the sliding devices, thereby meeting different needs.

It should be noted that the examples shown in FIGs. 1 and 6-9 and above descriptions thereof are merely provided for the purposes of illustration, and not intended to limit the scope of the present disclosure. For example, similar to the first supporting member, the second supporting member and/or the third supporting member may have a frame structure or a sliding device structure.

FIG. 10 is a schematic module diagram illustrating an exemplary medical system 100 according to some embodiments of the present disclosure.

As illustrated in FIG. 10, the medical system 100 may include a medical device 101, a couch 102, and a controller 103. The medical device 101 may be a medical device (e.g., any one of the medical devices 10, 60, 70, 80, and 90) described in the embodiments of the present disclosure. The couch 102 may be configured to support a patient (e.g., the patient 20 in FIG. 1). The controller 103 may be configured to process data related to the medical system 100.

In some embodiments, the medical device 101 may further include a first scanning device (e.g., the first scanning device 18) and/or a second scanning device (e.g., the second scanning device 23 or 68). The first scanning device may include a first scanning ray emitter configured to emit first scanning rays and a first scanning ray receiver configured to receive the first scanning rays emitted by the first scanning ray emitter. The first scanning ray emitter and the first scanning ray receiver may be rotatably mounted on a first support member around a second rotation axis synchronously. The second scanning device may include a second scanning ray emitter configured to emit second scanning rays and a second scanning ray receiver configured to receive the second scanning rays emitted by the second scanning ray emitter. The second scanning ray emitter and the second scanning ray receiver may be fixedly mounted on the first support member.

In some embodiments, the controller 103 may be configured to determine a region to be treated for the patient; control the couch 102 to move such that an isocenter of the medical device 101 may be located in the region to be treated; control a treatment device to rotate around the second rotation axis to adjust an irradiation angle of the treatment rays to be emitted by the treatment ray emitter; and control the first support member to rotate around the first rotation axis while controlling the treatment device to emit the treatment rays for performing radiotherapy.

The region to be treated refers to a region that needs to be treated or scanned and imaged for the patient, i.e., a lesion site. In some embodiments, the region to be treated for the patient may be determined by pre-stored lesion data of the patient. In some embodiments, the region to be treated for the patient may be determined by inputting the lesion data of the patient into the medical system 100. In some embodiments, the controller 103 may be further configured to control the first scanning device and/or the second scanning device to scan and acquire a planning image of the patient, and determine the region to be treated for the patient based on the planning image. For example, target objects (e.g., various signs) may be arranged in the region to be treated for the patient, and the region to be treated for the patient may be determined by recognizing the target objects in the planning image. As another example, a medical worker may determine the region to be treated for the patient by performing operations such as clicking or circling the region to be treated for the patient in the planning image.

In some embodiments, positions of an isocenter point of the treatment device and an isocenter point of the scanning device may always be constant, so that the positions of the isocenter points may be stored in the controller. The controller 103 may obtain the positions of the isocenter points and the region to be treated, and control the couch to move until the isocenter points are located in the region to be treated. Merely by way of example, the couch may have a fixed initial position, and a position of the patient relative to the couch may be basically fixed. After the region to be treated of the patient is determined, the position of the region to be treated relative to the couch be determined. The controller 103 may be in communicating connection with a movement system of the couch to send a movement instruction to the movement system of the couch, thereby controlling the couch to move such that the positions of the isocenter points are located in the region to be treated.

In some embodiments, due to a positioning error between the region to be treated and the isocenter points, a movement error may occur in the movement of the couch and a body displacement of the patient. Therefore, in some embodiments, in order to avoid the positioning error and the movement error of the medical system 100, the controller 103 may control the first scanning device and/or the second scanning device to acquire a preoperative guidance image before an irradiation angle is adjusted to ensure that the positions of the isocenter points are located in the region to be treated. In response to determining that the positions of the isocenter points are located in the region to be treated, the controller may control the treatment device to rotate around the second rotation axis to adjust the irradiation angle of the treatment rays emitted by the treatment ray emitter of the treatment device.

In some embodiments, when the treatment device is controlled to perform radiotherapy, the controller 103 may be further configured to control the first scanning device and/or the second scanning device to acquire a treatment image of the patient, and determine whether the treatment rays emitted by the treatment device need to be adjusted based on the treatment image. For example, a plurality of treatment images may be acquired during radiotherapy, and whether a movement amplitude of the region to be treated exceeds a threshold may be determined based on the plurality of treatment images. If the movement amplitude exceeds the threshold, the controller 103 may control the treatment device to stop emitting the treatment rays.
Alternatively, the controller 103 may redetermine an emission angle of the treatment ray based on a latest second scanning device, and control the treatment device to adjust the emission angle of the treatment device so that the treatment rays may always target the region to be treated. In some embodiments, the planning image may be acquired by the second scanning device, and the treatment image may be acquired by the first scanning device.

In some embodiments, the controller 103 may be implemented by a computing device. For example, the computing device may include a processor, a storage, an input/output (I/O), and a communication port. In some embodiments, the controller 103 may be part of the medical device 101.

The present disclosure further provides a radiotherapy method based on the medical device and the medical system provided in the foregoing embodiments. The radiotherapy method may include the following steps: causing a patient to lie on a couch; then determining a region to be treated for the patient and a position of an isocenter of the medical device; after the region to be treated for the patient is determined, controlling the couch to move so that the isocenter of the medical device is located in the region treated; and adjusting an irradiation angle of treatment rays emitted by a treatment ray emitter of a treatment device by controlling the treatment device to rotate around a second rotation axis based on a position and a range of the region to be treated. In some embodiments, before the irradiation angle of the treatment rays is adjusted, the first scanning device and/or the second scanning device may be controlled to acquire a preoperative guidance image to determine that the position of the isocenter is located in the region treated. After the irradiation angle of the treatment rays is adjusted, a first support member may be controlled to rotate around a first rotation axis while controlling the treatment device to perform radiotherapy.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure, and are within the scope of the exemplary embodiments of this disclosure.

Moreover, certain terminology has been used to describe embodiments of the present disclosure. For example, the terms "one embodiment," "an embodiment," and/or "some embodiments" may mean that a particular feature, structure or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present disclosure.

Aspects of the present disclosure may be implemented entirely hardware, entirely software (including firmware, resident software, microcode, etc.) or combining software and hardware implementation that may all generally be referred to herein as a "unit," "module," or "system." Furthermore, aspects of the present disclosure may take the form of a computer program product embodied in one or more computer readable media having computer readable program code embodied thereon.

Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications that are within the scope of the disclosed embodiments. For example, although the implementation of various components described above may be embodied in a hardware device, it may also be implemented as a software only solution, for example, an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This method of disclosure, however, is not to be interpreted as reflecting an intention that the claimed object matter requires more features than are expressly recited in each claim. Rather, inventive embodiments lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate ±1%, ±5%, ±10%, or ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

In closing, it is to be understood that the embodiments of the application disclosed herein are illustrative of the embodiments of the application. Other modifications that may be employed may be within the scope of the application. Thus, by way of example, but not of limitation, alternative configurations of the embodiments of the application may be utilized in accordance with the teachings herein. Accordingly, embodiments of the present application are not limited to that precisely as shown and described.

## Claims

1. A medical device (10, 60, 70, 80, 90), comprising:
a gantry (11, 61);
a first support member (12, 62, 91) rotatably mounted on the gantry (11, 61) around a first rotation axis (15);
a treatment device (17, 67) including a treatment ray emitter (171, 671) configured to emit treatment rays and a treatment ray receiver (172, 672) configured to receive the treatment rays, wherein the treatment ray emitter (171, 671) and the treatment ray receiver (172, 672) are rotatably mounted on the first support member (12, 62, 91) around a second rotation axis (16), the treatment ray emitter (171, 671) and the treatment ray receiver (172, 672) configured to
rotate synchronously, and the first rotation axis (15) intersects with the second rotation axis (16);
a second support member (13, 63) and a third support member (14, 64), **characterized in that** the second support member (13, 63) and the third support member (14, 64) are spaced apart from each other along an extension direction of the second rotation axis (16), the second support member (13, 63) and the third support member (14, 64) are rotatably mounted on the first support member (12, 62) around the second rotation axis (16), and the second support member (13, 63) and the third support member (14, 64) rotate synchronously,
the treatment ray emitter (171, 671) is mounted on one of the second support member (13, 63) and the third support member (14, 64), the treatment ray receiver (172, 672) is mounted on the other of the second support member (13, 63) and the third support member (14, 64),
the treatment ray receiver (172, 672) is mounted on a transmission path of the treatment rays emitted by the treatment ray emitter (171, 671).

2. The medical device (10, 70, 80) of claim 1, wherein the first support member (12) includes an annular structure, the gantry (11) includes an accommodating hole (111),
the annular structure is mounted within the accommodating hole (111), and
the second support member (13) and the third support member (14) are connected with an inner ring of the first support member (12).

3. The medical device (80) of claim 1 or 2, the second support member (13) is rotatably mounted on the first support member (12) around a third rotation axis, the third rotation axis is parallel to an extension direction of an intersection line (21) between the second support member (13) and the first support member (12),
the third support member (14) is rotatably mounted on the first support member (12) around a fourth rotation axis, the fourth rotation axis is parallel to an extension direction of an intersection line (22) between the third support member (14) and the first support member (12), and
the third rotation axis is parallel to the fourth rotation axis.

4. The medical device (10, 70, 80, 90) of any one of claims 1 to 3, further comprising:
a first scanning device (18) including a first scanning ray emitter (181) configured to emit first scanning rays and a first scanning ray receiver (182) configured to receive the first scanning rays, wherein the first scanning ray emitter (181) and the first scanning ray receiver (182) are rotationally mounted on the first support member (12) around the second rotation axis (16), and the first scanning ray emitter (181) and the first scanning ray receiver (182) rotate synchronously, and
an isocenter of the treatment device (17) coincides with an isocenter of the first scanning device (18).

5. The medical device (10, 80, 90) of claim 4, wherein
the first scanning ray emitter (181) is mounted on one of the second support member (13) and the third support member (14), and the first scanning ray receiver (182) is mounted on the other of the second support member (13) and the third support member (14), and
the first scanning ray receiver (182) is mounted on a transmission path of the first scanning rays emitted by the first scanning ray emitter (181).

6. The medical device (60, 70) of any one of claims 1 to 5, further comprising a second scanning device (23, 68) including a second scanning ray emitter (231, 681) configured to emit second scanning rays and a second scanning ray receiver (232, 682) configured to receive the second scanning rays, wherein the second scanning ray emitter (231, 681) and the second scanning ray receiver (232, 682) are fixedly mounted on the first support member (12, 62).

7. The medical device (60, 70) of claim 6, wherein the second scanning ray emitter (231, 681) and the second scanning ray receiver (232, 682) are mounted on different sides of a plane defined by the first rotation axis (15) and the second rotation axis (16).

8. The medical device (60) of claim 6 or7, wherein the gantry (61) includes a first gantry component (612) and a second gantry component (613), and the first gantry component (612) and the second gantry component (613) are spaced apart from each other along an extension direction of the first rotation axis (15), and
two ends of the first support member (62) along the extension direction of the first rotation axis (15) are rotationally connected with the first gantry component (612) and the second gantry component (613), respectively.

9. The medical device (60) of claim 8, wherein the first support member (62) includes a first support end portion (621), a second support end portion (622), and a connecting portion (623),
the connecting portion (623) is located between and connects the first support end portion (621) and the second support end portion (622), the first support end portion (621) is rotationally connected with the first gantry component (612), the second support end portion (622) is rotationally connected with the second gantry component (613),
the second support member (63) and the third support member (64) are rotatably mounted on the connecting portion (623) around the second rotation axis (16), and
the second scanning ray emitter (681) and the second scanning ray receiver (682) are fixed on the connecting portion (623).

10. The medical device (10, 60, 70, 80, 90) of any one of claims 1 to 9, wherein the first rotation axis (15) is perpendicular to the second rotation axis (16), and an intersection point between the first rotation axis (15) and the second rotation axis (16) coincides with an isocenter of the medical device (10, 60, 70).

11. The medical device (90) of claim 1, wherein
the medical device (90) further comprises a slide rail (24) arranged along a circumferential direction of the gantry (11), an axial direction of the slide rail (24) coincides with the first rotation axis (15),
the first support member (12) includes a first sliding device (911) and a second sliding device (912), the first sliding device (911) is configured to slidably mount the treatment ray emitter (171) in the slide rail (24), the second sliding device (912) is configured to slidably mount the treatment ray receiver (172) in the slide rail (24), and
the first sliding device (911) and the second sliding device (912) are symmetrically arranged on two sides of the first rotation axis (15).

12. The medical device (90) of claim 11, wherein
the first sliding device (911) includes a first pulley (9111) mounted in the slide rail (24) and a first connecting rod (9112) configured to connect the first pulley (9111) and the treatment ray emitter (171), a length of the first connecting rod (9112) is adjustable for adjusting a distance from the treatment ray emitter (171) to the gantry (11), and
the second sliding device (912) includes a second pulley (9121) mounted in the slide rail (24) and a second connecting rod (9122) configured to connect the second pulley (9121) and the treatment ray receiver (171), a length of the second connecting rod (9122) is adjustable for adjusting a distance from the treatment ray receiver (172) to the gantry (11).

13. The medical device (70) of any one of claims 1 to 3, further comprising:
a first scanning device (18) including a first scanning ray emitter (181) configured to emit first scanning rays and a first scanning ray receiver (182) configured to receive the first scanning rays, wherein the first scanning ray emitter (181) and the first scanning ray receiver (182) are rotatably mounted on the first support member (12) around the second rotation axis (16), and the first scanning ray emitter (181) and the first scanning ray receiver (182) rotate synchronously; and
a second scanning device (23) including a second scanning ray emitter (231) configured to emit second scanning rays and a second scanning ray receiver (232) configured to receive the second scanning rays, the second scanning ray emitter (231) and the second scanning ray receiver (232) being fixedly mounted on the first support member (12); wherein
an emission period of the first scanning rays at least partially coincides with an emission period of the treatment rays; and
an emission period of the second scanning rays does not coincide with the emission period of the treatment rays.

14. The medical device (90) of claim 13, wherein the emission period of the second radiation rays is before the emission period of the treatment rays, the second scanning device (23) is configured to acquire a planning image, and the dose of the second scanning rays is higher than the dose of the first scanning rays.

15. The medical device of claim 14, wherein the first scanning device (18) is a digital radiographic (DR) device, and the second scanning device (23) is a computed tomographic (CT) device.

## Patentansprüche

1. Medizinische Vorrichtung (10, 60, 70, 80, 90), umfassend:
ein Gestell (11, 61);
ein erstes Stützelement (12, 62, 91), das drehbar auf dem Gestell (11, 61) um eine erste Drehachse (15) montiert ist;
eine Behandlungsvorrichtung (17, 67), die einen Behandlungsstrahlenemitter (171, 671), der zum Aussenden von Behandlungsstrahlen konfiguriert ist, und einen Behandlungsstrahlenempfänger (172, 672), der zum Empfangen der Behandlungsstrahlen konfiguriert ist, einschließt, wobei der Behandlungsstrahlenemitter (171, 671) und der Behandlungsstrahlenempfänger (172, 672) drehbar auf dem ersten Stützelement (12, 62, 91) um eine zweite Drehachse (16) montiert sind, der Behandlungsstrahlenemitter (171, 671) und der Behandlungsstrahlenempfänger (172, 672) konfiguriert sind, um sich synchron zu drehen, und die erste Drehachse (15) die zweite Drehachse (16) schneidet;
ein zweites Stützelement (13, 63) und ein drittes Stützelement (14, 64), **dadurch gekennzeichnet, dass**
das zweite Stützelement (13, 63) und das dritte Stützelement (14, 64) in einer Verlängerungsrichtung der zweiten Drehachse (16) voneinander beabstandet sind, das zweite Stützelement (13, 63) und das dritte Stützelement (14, 64) drehbar auf dem ersten Stützelement (12, 62) um die zweite Drehachse (16) montiert sind und das zweite Stützelement (13, 63) und das dritte Stützelement (14, 64) sich synchron drehen,
der Behandlungsstrahlenemitter (171, 671) an einem von dem zweiten Stützelement (13, 63) und dem dritten Stützelement (14, 64) montiert ist, der Behandlungsstrahlenempfänger (172, 672) auf dem anderen von dem zweiten Stützelement (13, 63) und dem dritten Stützelement (14, 64) montiert ist,
der Behandlungsstrahlenempfänger (172, 672) auf einem Übertragungspfad der vom Behandlungsstrahlenemitter (171, 671) ausgesendeten Behandlungsstrahlen montiert ist.

2. Medizinische Vorrichtung (10, 70, 80) nach Anspruch 1, wobei das erste Stützelement (12) eine ringförmige Struktur einschließt und das Gestell (11) eine Aufnahmeöffnung (111) einschließt,
die ringförmige Struktur in der Aufnahmebohrung (111) montiert ist, und
das zweite Stützelement (13) und das dritte Stützelement (14) mit einem inneren Ring des ersten Stützelements (12) verbunden sind.

3. Medizinische Vorrichtung (80) nach Anspruch 1 oder 2, wobei das zweite Stützelement (13) drehbar auf dem ersten Stützelement (12) um eine dritte Drehachse montiert ist, die dritte Drehachse parallel zu einer Verlängerungsrichtung einer Schnittlinie (21) zwischen dem zweiten Stützelement (13) und dem ersten Stützelement (12) verläuft,
das dritte Stützelement (14) drehbar auf dem ersten Stützelement (12) um eine vierte Drehachse montiert ist, die vierte Drehachse parallel zu einer Verlängerungsrichtung einer Schnittlinie (22) zwischen dem dritten Stützelement (14) und dem ersten Stützelement (12) verläuft, und
die dritte Drehachse parallel zur vierten Drehachse verläuft.

4. Medizinische Vorrichtung (10, 70, 80, 90) nach einem der Ansprüche 1 bis 3, weiter umfassend:
eine erste Abtastvorrichtung (18), die einen ersten Abtaststrahlenemitter (181), der zum Aussenden erster Abtaststrahlen konfiguriert ist, und einen ersten Abtaststrahlenempfänger (182), der zum Empfangen der ersten Abtaststrahlen konfiguriert ist, einschließt, wobei der erste Abtaststrahlenemitter (181) und der erste Abtaststrahlenempfänger (182) drehbar auf dem ersten Stützelement (12) um die zweite Drehachse (16) montiert sind und der erste Abtaststrahlenemitter (181) und der erste Abtaststrahlenempfänger (182) sich synchron drehen, und
ein Isozentrum der Behandlungsvorrichtung (17) mit einem Isozentrum der ersten Abtastvorrichtung (18) zusammenfällt.

5. Medizinische Vorrichtung (10, 80, 90) nach Anspruch 4, wobei
der erste Abtaststrahlemitter (181) auf einem der von dem zweiten Stützelement (13) und dem dritten Stützelement (14) montiert ist und der erste Abtaststrahlempfänger (182) auf dem anderen von dem zweiten Stützelement (13) und dem dritten Stützelement (14) montiert ist, und
der erste Abtaststrahlempfänger (182) auf einem Übertragungspfad der ersten Abtaststrahlen montiert ist, die vom ersten Abtaststrahlemitter (181) ausgesendet werden.

6. Medizinische Vorrichtung (60, 70) nach einem der Ansprüche 1 bis 5, weiter umfassend eine zweite Abtastvorrichtung (23, 68), die einen zweiten Abtaststrahlenemitter (231, 681), der zum Aussenden zweiter Abtaststrahlen konfiguriert ist, und einen zweiten Abtaststrahlenempfänger (232, 682), der zum Empfangen der zweiten Abtaststrahlen konfiguriert ist, einschließt, wobei der zweite Abtaststrahlenemitter (231, 681) und der zweite Abtaststrahlenempfänger (232, 682) fest auf dem ersten Stützelement (12, 62) montiert sind.

7. Medizinische Vorrichtung (60, 70) nach Anspruch 6, wobei der zweite Abtaststrahlemitter (231, 681) und der zweite Abtaststrahlempfänger (232, 682) auf verschiedenen Seiten einer Ebene angeordnet sind, die durch die erste Drehachse (15) und die zweite Drehachse (16) definiert ist.

8. Medizinische Vorrichtung (60) nach Anspruch 6 oder 7, wobei das Gestell (61) ein erstes Gestellbauteil (612) und ein zweites Gestellbauteil (613) einschließt und das erste Gestellbauteil (612) und das zweite Gestellbauteil (613) entlang einer Verlängerungsrichtung der ersten Drehachse (15) voneinander beabstandet sind, und
die beiden Enden des ersten Stützelements (62) entlang der Verlängerungsrichtung der ersten Drehachse (15) mit dem ersten Gestellbauteil (612) bzw. dem zweiten Gestellbauteil (613) drehbar verbunden sind.

9. Medizinische Vorrichtung (60) nach Anspruch 8, wobei das erste Stützelement (62) einen ersten Stützendabschnitt (621), einen zweiten Stützendabschnitt (622) und einen Verbindungsabschnitt (623) einschließt,
der Verbindungsabschnitt (623) sich zwischen dem ersten Stützendabschnitt (621) und dem zweiten Stützendabschnitt (622) befindet und diese verbindet, der erste Stützendabschnnitt (621) drehbar mit dem ersten Gestellbauteil (612) verbunden ist, der zweite Stützendabschnnitt (622) drehbar mit dem zweiten Gestellbauteil (613) verbunden ist,
das zweite Stützelement (63) und das dritte Stützelement (64) drehbar auf dem Verbindungsabschnitt (623) um die zweite Drehachse (16) montiert sind, und
der zweite Abtaststrahlemitter (681) und der zweite Abtaststrahlempfänger (682) am Verbindungsabschnitt (623) befestigt sind.

10. Medizinische Vorrichtung (10, 60, 70, 80, 90) nach einem der Ansprüche 1 bis 9, wobei die erste Drehachse (15) senkrecht zur zweiten Drehachse (16) steht und ein Schnittpunkt zwischen der ersten Drehachse (15) und der zweiten Drehachse (16) mit einem Isozentrum der medizinischen Vorrichtung (10, 60, 70) zusammenfällt.

11. Medizinische Vorrichtung (90) nach Anspruch 1, wobei
die medizinische Vorrichtung (90) weiter eine Gleitschiene (24) umfasst, die in einer Umfangsrichtung des Gestells (11) angeordnet ist, eine axiale Richtung der Gleitschiene (24) mit der ersten Drehachse (15) zusammenfällt,
das erste Stützelement (12) eine erste Gleitvorrichtung (911) und eine zweite Gleitvorrichtung (912) einschließt, die erste Gleitvorrichtung (911) so konfiguriert ist, dass sie den Behandlungsstrahlenemitter (171) verschiebbar in der Gleitschiene (24) montiert, und die zweite Gleitvorrichtung (912) so konfiguriert ist, dass sie den Behandlungsstrahlenempfänger (172) verschiebbar in der Gleitschiene (24) montiert, und
die erste Gleitvorrichtung (911) und die zweite Gleitvorrichtung (912) symmetrisch auf zwei Seiten der ersten Drehachse (15) angeordnet sind.

12. Medizinische Vorrichtung (90) nach Anspruch 11, wobei
die erste Gleitvorrichtung (911) eine erste in der Gleitschiene (24) montierte Riemenscheibe (9111) und eine erste Verbindungsstange (9112) einschließt, die so konfiguriert ist, dass sie die erste Riemenscheibe (9111) mit dem Behandlungsstrahlenemitter (171) verbindet, eine Länge der ersten Verbindungsstange (9112) verstellbar ist, um einen Abstand zwischen dem Behandlungsstrahlenemitter (171) und dem Gestell (11) anzupassen, und
die zweite Gleitvorrichtung (912) eine zweite Riemenscheibe (9121), die in der Gleitschiene (24) montiert ist, und eine zweite Verbindungsstange (9122) einschließt, die so konfiguriert ist, dass sie die zweite Riemenscheibe (9121) mit dem Behandlungsstrahlenempfänger (171) verbindet, eine Länge der zweiten Verbindungsstange (9122) verstellbar ist, um einen Abstand zwischen dem Behandlungsstrahlenempfänger (172) und dem Gestell (11) anzupassen.

13. Medizinische Vorrichtung (70) nach einem der Ansprüche 1 bis 3, weiter umfassend:
eine erste Abtastvorrichtung (18), die einen ersten Abtaststrahlenemitter (181), der zum Aussenden erster Abtaststrahlen konfiguriert ist, und einen ersten Abtaststrahlenempfänger (182), der zum Empfangen der ersten Abtaststrahlen konfiguriert ist, einschließt, wobei der erste Abtaststrahlenemitter (181) und der erste Abtaststrahlenempfänger (182) drehbar auf dem ersten Stützelement (12) um die zweite Drehachse (16) montiert sind und der erste Abtaststrahlenemitter (181) und der erste Abtaststrahlenempfänger (182) sich synchron drehen, und
eine zweite Abtastvorrichtung (23), die einen zweiten Abtaststrahlenemitter (231), der zum Aussenden zweiter Abtaststrahlen konfiguriert ist, und einen zweiten Abtaststrahlenempfänger (232), der zum Empfangen der zweiten Abtaststrahlen konfiguriert ist, einschließt, wobei der zweite Abtaststrahlenemitter (231) und der zweite Abtaststrahlenempfänger (232) fest auf dem ersten Stützelement (12) montiert sind; wobei
eine Emissionsperiode der ersten Abtaststrahlen zumindest teilweise mit einer Emissionsperiode der Behandlungsstrahlen zusammenfällt; und
eine Emissionsperiode der zweiten Abtaststrahlen nicht mit der Emissionsperiode der Behandlungsstrahlen zusammenfällt.

14. Medizinische Vorrichtung (90) nach Anspruch 13, wobei die Emissionsperiode der zweiten Strahlungsstrahlen vor der Emissionsperiode der Behandlungsstrahlen liegt, die zweite Abtastvorrichtung (23) zur Aufnahme eines Planungsbildes konfiguriert ist und die Dosis der zweiten Abtaststrahlen höher ist als die Dosis der ersten Abtaststrahlen.

15. Medizinische Vorrichtung nach Anspruch 14, wobei es sich bei der ersten Abtastvorrichtung (18) um eine digitale Röntgenvorrichtung (DR) handelt und bei der zweiten Abtastvorrichtung (23) um eine Computertomographievorrichtung (CT) handelt.

## Revendications

1. Dispositif médical (10, 60, 70, 80, 90), comprenant :
un portique (11, 61) ;
un premier élément de support (12, 62, 91) monté de manière rotative sur le portique (11, 61) autour d'un premier axe de rotation (15) ;
un dispositif de traitement (17, 67) incluant un émetteur de rayons de traitement (171, 671) configuré pour émettre des rayons de traitement et un récepteur de rayons de traitement (172, 672) configuré pour recevoir les rayons de traitement, dans lequel l'émetteur de rayons de traitement (171, 671) et le récepteur de rayons de traitement (172, 672) sont montés de manière rotative sur le premier élément de support (12, 62, 91) autour d'un deuxième axe de rotation (16), l'émetteur de rayons de traitement (171, 671) et le récepteur de rayons de traitement (172, 672) sont configurés pour tourner de manière synchrone, et le premier axe de rotation (15) croise le deuxième axe de rotation (16);
un deuxième élément de support (13, 63) et un troisième élément de support (14, 64), **caractérisé en ce que**
le deuxième élément de support (13, 63) et le troisième élément de support (14, 64) sont espacés l'un de l'autre le long d'une direction d'extension du deuxième axe de rotation (16), le deuxième élément de support (13, 63) et le troisième élément de support (14, 64) sont montés de manière rotative sur le premier élément de support (12, 62) autour du deuxième axe de rotation (16), et le deuxième élément de support (13, 63) et le troisième élément de support (14, 64) tournent de manière synchrone,
l'émetteur de rayons de traitement (171, 671) est monté sur un parmi le deuxième élément de support (13, 63) et le troisième élément de support (14, 64), le récepteur de rayons de traitement (172, 672) est monté sur l'autre parmi le deuxième élément de support (13, 63) et le troisième élément de support (14, 64),
le récepteur de rayons de traitement (172, 672) est monté sur un chemin de transmission des rayons de traitement émis par l'émetteur de rayons de traitement (171, 671).

2. Dispositif médical (10, 70, 80) selon la revendication 1, dans lequel le premier élément de support (12) inclut une structure annulaire, le portique (11) inclut un trou d'accueil (111),
la structure annulaire est montée dans le trou d'accueil (111), et
le deuxième élément de support (13) et le troisième élément de support (14) sont reliés à un anneau intérieur du premier élément de support (12).

3. Dispositif médical (80) selon la revendication 1 ou 2, le deuxième élément de support (13) est monté de manière rotative sur le premier élément de support (12) autour d'un troisième axe de rotation, le troisième axe de rotation est parallèle à une direction d'extension d'une ligne d'intersection (21) entre le deuxième élément de support (13) et le premier élément de support (12),
le troisième élément de support (14) est monté de manière rotative sur le premier élément de support (12) autour d'un quatrième axe de rotation, le quatrième axe de rotation est parallèle à une direction d'extension d'une ligne d'intersection (22) entre le troisième élément de support (14) et le premier élément de support (12), et
le troisième axe de rotation est parallèle au quatrième axe de rotation.

4. Dispositif médical (10, 70, 80, 90) selon l'une quelconque des revendications 1 à 3, comprenant en outre :
un premier dispositif de balayage (18) incluant un premier émetteur de rayons de balayage (181) configuré pour émettre des premiers rayons de balayage et un premier récepteur de rayons de balayage (182) configuré pour recevoir les premiers rayons de balayage, dans lequel le premier émetteur de rayons de balayage (181) et le premier récepteur de rayons de balayage (182) sont montés de manière rotative sur le premier élément de support (12) autour du deuxième axe de rotation (16), et le premier émetteur de rayons de balayage (181) et le premier récepteur de rayons de balayage (182) tournent de manière synchrone, et
un isocentre du dispositif de traitement (17) coïncide avec un isocentre du premier dispositif de balayage (18).

5. Dispositif médical (10, 80, 90) selon la revendication 4, dans lequel
le premier émetteur de rayons de balayage (181) est monté sur un parmi le deuxième élément de support (13) et le troisième élément de support (14), et le premier récepteur de rayons de balayage (182) est monté sur l'autre parmi le deuxième élément de support (13) et le troisième élément de support (14), et
le premier récepteur de rayons de balayage (182) est monté sur un chemin de transmission des premiers rayons de balayage émis par le premier émetteur de rayons de balayage (181).

6. Dispositif médical (60, 70) selon l'une quelconque des revendications 1 à 5, comprenant en outre un second dispositif de balayage (23, 68) incluant un second émetteur de rayons de balayage (231, 681) configuré pour émettre des seconds rayons de balayage et un second récepteur de rayons de balayage (232, 682) configuré pour recevoir les seconds rayons de balayage, dans lequel le second émetteur de rayons de balayage (231, 681) et le second récepteur de rayons de balayage (232, 682) sont montés de manière fixe sur le premier élément de support (12, 62).

7. Dispositif médical (60, 70) selon la revendication 6, dans lequel le second émetteur de rayons de balayage (231, 681) et le second récepteur de rayons de balayage (232, 682) sont montés sur des côtés différents d'un plan défini par le premier axe de rotation (15) et le deuxième axe de rotation (16).

8. Dispositif médical (60) selon la revendication 6 ou 7, dans lequel le portique (61) inclut un premier composant de portique (612) et un second composant de portique (613), et le premier composant de portique (612) et le second composant de portique (613) sont espacés l'un de l'autre le long d'une direction d'extension du premier axe de rotation (15), et
deux extrémités du premier élément de support (62) le long de la direction d'extension du premier axe de rotation (15) sont reliées de manière rotative au premier composant de portique (612) et au second composant de portique (613), respectivement.

9. Dispositif médical (60) selon la revendication 8, dans lequel le premier élément de support (62) inclut une première partie d'extrémité de support (621), une seconde partie d'extrémité de support (622) et une partie de liaison (623),
la partie de liaison (623) est située entre et relie la première partie d'extrémité de support (621) et la seconde partie d'extrémité de support (622), la première partie d'extrémité de support (621) est reliée de manière rotative au premier composant de portique (612), la seconde partie d'extrémité de support (622) est reliée de manière rotative au second composant de portique (613),
le deuxième élément de support (63) et le troisième élément de support (64) sont montés de manière rotative sur la partie de liaison (623) autour du deuxième axe de rotation (16), et
le second émetteur de rayons de balayage (681) et le second récepteur de rayons de balayage (682) sont fixés sur la partie de liaison (623).

10. Dispositif médical (10, 60, 70, 80, 90) selon l'une quelconque des revendications 1 à 9, dans lequel le premier axe de rotation (15) est perpendiculaire au deuxième axe de rotation (16), et un point d'intersection entre le premier axe de rotation (15) et le deuxième axe de rotation (16) coïncide avec un isocentre du dispositif médical (10, 60, 70).

11. Dispositif médical (90) selon la revendication 1, dans lequel
le dispositif médical (90) comprend en outre un rail de coulissement (24) agencé le long d'une direction circonférentielle du portique (11), une direction axiale du rail de coulissement (24) coïncide avec le premier axe de rotation (15),
le premier élément de support (12) inclut un premier dispositif coulissant (911) et un second dispositif coulissant (912), le premier dispositif coulissant (911) est configuré pour monter de manière coulissante l'émetteur de rayons de traitement (171) dans le rail de coulissement (24), le second dispositif coulissant (912) est configuré pour monter de manière coulissante le récepteur de rayons de traitement (172) dans le rail de coulissement (24), et
le premier dispositif coulissant (911) et le second dispositif coulissant (912) sont agencés symétriquement sur deux côtés du premier axe de rotation (15).

12. Dispositif médical (90) selon la revendication 11, dans lequel
le premier dispositif coulissant (911) inclut une première poulie (9111) montée dans le rail de coulissement (24) et une première bielle (9112) configurée pour relier la première poulie (9111) et l'émetteur de rayons de traitement (171), une longueur de la première bielle (9112) est réglable pour régler une distance de l'émetteur de rayons de traitement (171) au portique (11), et
le second dispositif coulissant (912) inclut une seconde poulie (9121) montée dans le rail de coulissement (24) et une seconde bielle (9122) configurée pour relier la seconde poulie (9121) et le récepteur de rayons de traitement (171), une longueur de la seconde bielle (9122) est réglable pour régler une distance du récepteur de rayons de traitement (172) au portique (11).

13. Dispositif médical (70) selon l'une quelconque des revendications 1 à 3, comprenant en outre :
un premier dispositif de balayage (18) incluant un premier émetteur de rayons de balayage (181) configuré pour émettre des premiers rayons de balayage et un premier récepteur de rayons de balayage (182) configuré pour recevoir les premiers rayons de balayage, dans lequel le premier émetteur de rayons de balayage (181) et le premier récepteur de rayons de balayage (182) sont montés de manière rotative sur le premier élément de support (12) autour du deuxième axe de rotation (16), et le premier émetteur de rayons de balayage (181) et le premier récepteur de rayons de balayage (182) tournent de manière synchrone ; et
un second dispositif de balayage (23) incluant un second émetteur de rayons de balayage (231) configuré pour émettre des seconds rayons de balayage et un second récepteur de rayons de balayage (232) configuré pour recevoir les seconds rayons de balayage, le second émetteur de rayons de balayage (231) et le second récepteur de rayons de balayage (232) étant montés de manière fixe sur le premier élément de support (12) ; dans lequel
une période d'émission des premiers rayons de balayage coïncide au moins partiellement avec une période d'émission des rayons de traitement ; et
une période d'émission des seconds rayons de balayage ne coïncide pas avec la période d'émission des rayons de traitement.

14. Dispositif médical (90) selon la revendication 13, dans lequel la période d'émission des seconds rayons de rayonnement est antérieure à la période d'émission des rayons de traitement, le second dispositif de balayage (23) est configuré pour acquérir une image de planification, et la dose des seconds rayons de balayage est supérieure à la dose des premiers rayons de balayage.

15. Dispositif médical selon la revendication 14, dans lequel le premier dispositif de balayage (18) est un dispositif de radiographie numérique (DR) et le second dispositif de balayage (23) est un dispositif de tomographie assistée par ordinateur (CT).
